# EUROPEAN PATENT SPECIFICATION

(11) **EP 0 877 580 B1**
(45) Date of publication and mention of the grant of the patent: **18.05.2005**
(21) Application number: 97909519.7
(22) Date of filing: 18.09.1997
(51) Int. Cl.: A61M 5/142, A61L 31/00, C08L 75/04, A61L 31/10

(54) **MEDICATION INFUSION PUMP WITH PROTEIN STABILIZED SURFACE COATING**
PUMPE ZUR MEDIKAMENTÖSEN INFUSION MIT EINER DURCH EIN PROTEIN STABILISIERTEN BESCHICHTUNG
POMPE SERVANT DIFFUSER UN PRODUIT MEDICAMENTEUX ET COMPORTANT UN REVETEMENT DE SURFACE CONTRE LES DEPOTS PROTEINES

(30) Priority: 01.11.1996 US 742377
(43) Date of publication of application: 18.11.1998
(73) Proprietor: Medtronic MiniMed, Inc., Northridge, CA 91325-1219 (US)
(72) Inventor: VAN ANTWERP, William, P., Valencia, CA 91355 (US); GULATI, Poonam, S., La Canada, CA 91011 (US); DECKER, Christian, C., San Diego, CA 92116 (US); ADOMIAN, Gerald, E., Los Angeles, CA 90049 (US); DY, Norman, V., Daly City, CA 94014 (US)
(74) Representative: Goldin, Douglas Michael
(86) International application number: PCT/IB1997/001375
(87) International publication number: WO 1998/019627

(56) References cited:
- WO-A-89/09246
- WO-A-93/10827
- US-A- 4 569 641
- US-A- 4 769 013
- US-A- 4 950 256
- US-A- 5 019 260
- US-A- 5 077 210
- US-A- 5 330 911
- US-A- 5 527 618
- FEINGOLD V ET AL: "EFFECT OF CONTACT MATERIAL ON VIBRATION-INDUCED INSULIN AGGREGATION" DIABETOLOGIA, vol. 27, no. 3, 1984, pages 373-378, XP001056926 ISSN: 0012-186X

## Description

### BACKGROUND OF THE INVENTION

This invention relates generally to medical devices, in particular to medication infusion pumps, particularly of the type adapted for implantation directly into the body of a patient and for programmed operation to deliver medication to the patient. More particularly, this invention relates to an improved medication infusion pump having one or more internal hydrophilic surfaces to minimize or eliminate accumulation of medication deposits from complex protein-based medications.

Medication infusion pumps are generally known in the art for use in delivering a selected medication to a patient in a scheduled or preprogrammed manner. In recent years, infusion pumps have been developed in compact form and adapted for direct implantation into a body of a patient, to deliver a specific medication such as insulin to the patient in discrete doses over an extended time period. An implantable infusion pump of this general type includes an internal medication chamber for receiving and storing a supply of the selected medication in liquid form, in combination with a miniature pump mechanism and associated programmable control means for operating the pump mechanism to deliver discrete doses of the medication from the internal storage chamber and through a catheter to the patient. For one illustrative example of an implantable medication infusion pump of this general type see, U.S. Pat. No. 4,573,994, to Fischell et al.

The internal pump mechanism typically comprises an electromagnetically driven pulsatile pump having a solenoid operated piston mounted for reciprocation within a cylinder to draw medication from the internal storage chamber, and to deliver such medication through the catheter to the patient. The pulsatile pump operates in conjunction with an inlet check valve having a spring-loaded valve member movable between open and closed positions with respect to an annular valve seat. The valve member and valve seat are normally constructed from biocompatable and relatively inert materials, such as a movable valve disk of a silicone elastomer material and a rigid annular valve seat defined at the end of a ferrule formed of a titanium or titanium alloy. For examples of pulsatile pump mechanisms used in implantable infusion pumps, see U.S. Pat. No. 4,568,250, to Falk et al.; U.S. Pat. No. 4,569,641 to Falk et al.; U.S. Pat. No. 4,636,150, to Falk et al.; and U.S. Pat. No. 4,714,234, to Falk et al.

Implantable medication infusion pumps of the general type described above have been used to deliver a wide variety of drugs to a patient. Such medications or drugs include, for example, insulin, baclofen, morphine, various antibiotics, and a number of chemotherapeutic agents. As protein-based medications become more prevalent, problems arise in reliable long-term administration of the medication to a patient. More specifically, regular drug injections are not optimal to achieve relatively constant blood concentration levels. Many of the newer medications comprise relatively complex protein-based substances having a high molecular weight, such that subcutaneous drug delivery can be problematic due to decreased or minimal uptake by either the capillary or omental systems. Implantable pumps for intraperitoneal or intravascular delivery resolve many of these concerns, particularly wherein the medication needs to be delivered to the patient on a regular basis for a prolonged period of time, such as one year or longer.

One problem encountered with implantable medication infusion pumps is that internal pump surfaces are typically constructed from titanium or similar metal materials. Such metallic surfaces have oxide coatings that render them hydrophobic, with typical free surface energies on the order of about 40 dyne/cm². At this low free surface energy, protein-based medications such as insulin can be adsorbed quite readily and can easily denature on the pump surfaces. Once denaturing occurs, the protein-based substance can aggregate to a form that is generally not bio-available to the patient and may in some cases lead to undesired immunological response.

Feingold *et al* (Diabetologia (1984) 27: 373-378) describes tests for insulin aggregation, and suggests that hydrophilic contact materials appeared more compatible with insulin stability than hydrophobic materials.

The present invention provides an improved medical device such as a medication infusion pump having an internal surface coating or coatings that are hydrophilic, resulting in device surfaces that are highly stable in the presence of complex protein-based medications.

### SUMMARY OF THE INVENTION

In accordance with the invention, a medical device such as a medication infusion pump is constructed with one or more internal hydrophilic surfaces for contacting complex protein based medications delivered by the pump to a patient. The use of such hydrophilic internal pump surfaces is particularly advantageous in a medication infusion pump of the type adapted for implantation into the body of a patient, and for programmable or otherwise regulated delivery of the medication to the patient over a prolonged period of time. The hydrophilic pump surfaces have been found to be particularly stable in the presence of protein based medications, thereby minimizing or eliminating undesired denaturing of the medication and/or related accumulation of aggregative deposits.

Accordingly, the invention provides a medical device having a surface contactable by a protein based medication, characterised in that a hydrophilic surfactant is covalently attached to said surface such that said surface has a surface contact angle less than 45 degrees, wherein said hydrophilic surfactant is a block ethylene/propylene copolymer.

Other features and advantages of the present invention will become more apparent from the following detailed description, taken in conjunction with the accompanying drawings which illustrate, by way of example, the principles of the invention.

### BRIEF DESCRIPTION OF THE DRAWINGS

The accompanying drawings illustrate the invention. In such drawings:
FIGURE I is a perspective view depicting a typical implantable medication infusion pump; and

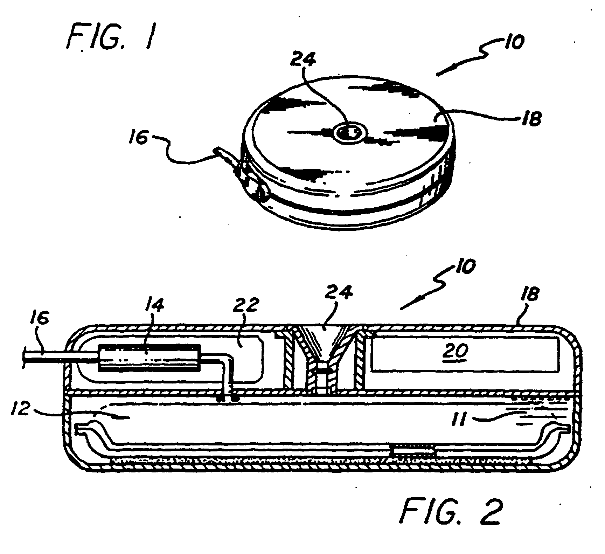

FIGURE 2 is an enlarged and somewhat schematic vertical sectional view of the pump of FIG. 1, and illustrating an internal pump mechanism for delivering medication from a medication storage reservoir to a patient.

### DETAILED DESCRIPTION OF THE PREFERRED EMBODIMENTS

As shown in the exemplary drawings, an implantable medication infusion pump referred to generally by the reference numeral 10 is provided for use in administering a selected medication to a patient in a controlled, preprogrammed manner. In accordance with the invention, the medication infusion pump 10 includes one or more internal pump surfaces bearing a hydrophilic coating or otherwise treated to exhibit hydrophilic characteristics, which have been found to substantially reduce or eliminate accumulation of undesired medication deposits particularly when the pump is used to deliver complex protein based medications such as insulin and the like. The hydrophilic internal pump surfaces have been found to significantly reduce and/or eliminate undesired adsorption of proteins on internal pump surfaces.

The medication infusion pump 10 shown in the illustrative drawings has a generally conventional overall construction and operation for delivering a selected medication 11 from an internal medication chamber or reservoir 12 (FIG. 2) through a catheter 16 to the patient. Medication delivery is accomplished by a miniature pump mechanism 14. In accordance with the present invention, surfaces of the medication chamber 12 and/or internal surfaces of the pump mechanism 14 include hydrophilic coatings to prevent adsorption of proteins and resultant accumulation of protein based medication deposits thereon.

FIGURES 1 and 2 show the medication infusion pump 10 in the form of a small substantially self-contained unit adapted for direct implantation into the body of a patient. The pump 10 comprises an hermetically sealed pump housing or case 18 formed from a biocompatable material, such as titanium or titanium alloy. The pump housing defines the internal medication reservoir 12 for receiving and storing the supply of the selected medication 11 in liquid form, such as insulin for a diabetic patient. The pump housing 18 encases the intemal pump mechanism 14 in combination with electronic control circuitry 20 and a battery 22 for periodically operating the pump mechanism 14 to deliver medication doses via the catheter 16 to the patient. The control circuitry 20 is suitably preprogrammed to deliver the medication in accordance with individual patient need. An inlet or refill fitting 24 on the pump housing 18 is adapted to receive a hypodermic needle (not shown) to permit transcutaneous refilling of the medication chamber 12 without requiring surgical access to the infusion pump 10.

For a more detailed description of the overall construction and operation of implantable infusion pumps of this general type, see U.S. Patent 4,373,527 and 4,573,994. For a more detailed description of the construction and operation of the miniature pump mechanism 14, see U.S. Patents 4,568,520; 4,569,241; 4,636,150; and 4,714,234.

The adsorption and subsequent denaturation of the protein-based medication on a surface does not usually depend on the exact chemical nature of the surface but instead is functionally related to its surface free energy. Accordingly, the present invention relates to an infusion pump wherein key internal surfaces are coated to achieve a significant reduction in surface free energy. Several surface treatment methods can be used to accomplish this objective, including hydrophilic surfactants, covalent bonding, and direct modification of the pump surface.

In accordance with a preferred surface treatment and method, a hydrophilic surfactant is applied to the selected pump surface to significantly reduce adsorption of a protein-based medication such as insulin. Several hydrophilic surfactants are available for this purpose, including but not limited to Genapol, a block ethylene/propylene copolymer having a molecular weight of about 1800 Daltons, available from Hoechst Celanese Co. of Somerville, New Jersey.

As one example using a hydrophilic surfactant, a 1.0% solution of Genapol is prepared in isopropanol and then contacted with the selected titanium pump surface, such as the titanium surface of the medication reservoir 12 by filling the reservoir with the Genapol solution. The Genapol surfactant which is non-ionic in nature binds to the titanium surface, and the isopropanol solvent can be readily removed under mild conditions of heat and vacuum. After this drying step, the treated titanium surface is placed in a radio frequency (RF) chamber in the presence of oxygen, argon, or both, and 100-200 watts of RF power are applied to result in covalent attachment of the polymer to the titanium surface. Preferably, this process is repeated at least once. An exemplary RF chamber is available from Technics, Inc. of Newark, New Jersey. During the RF treatment step, the oxygen and/or argon plasma generates significant ultraviolet light which creates reactive polymer sites which then covalently attach to the surface. In the illustrative example, each RF step proceeded for about 10 minutes using an RF frequency of about 100 KHz.

After this surface treatment with the Genapol surfactant and plasma, as described above, the surface contact angle is less than 10 degrees as measured by direct contact angle measurement. In this regard, the contact angle of water is a measure of its hydrophilic characteristics. A low contact angle means that the surface is wetted, whereas a high contact angle means that the surface is non-wetted or hydrophobic. The contact angle of an untreated or uncoated titanium surface is about 72 degrees.

Insulin adsorption after the Genapol surface treatment is less than 0.1 microgram per square cm of the titanium surface, as compared to an adsorption of about 0.6 microgram per square cm for uncoated titanium. Similar surface treatments using other hydrophilic surfactants such as those identified above yield results of similar magnitude, although Genapol is believed to provide the best reduction in insulin adsorption.

A more robust coating can be obtained by covalant attachment of the coating material to the selected surface of the pump. There are several ways to covalently attach a hydrophilic coating to the pump surface. These include radiation, electron beam and photo induced grafting, polymerization chemical grafting and plasma deposition of polymers. In general, these methods involve an energy source and a monomer of the desired hydrophilic polymer. For example, acrylonitrile can be grafted onto a titanium surface by irradiation of acrylonitrile vapor in contact with the surface. The resulting polymer, polyacrylonitrile (PAN) has excellent hydrophilic properties with very minimal protein interaction with the surface. A wide variety of polymers can be produced in this manner, the only requirement being that the monomer be available in reasonable purity with enough vapor pressure to be reactive in the deposition system.

In addition to the polymerization systems described above, a covalent attachment of a polymer to the surface can be accomplished via a silane (or other coupling) agent. In this case, a silane coupling is reacted with the pump surface. One typical silane for this attachment might be dichloro methyl vinyl silane which when hydrolyzed in aqueous ethanol will bond tenaciously to a metal surface through either an O-Si or metal Si bond. The vinyl group of the silane can then be reacted with a variety of either polymers or monomers. In an example, a methacrylate terminated PEG can be reacted with the terminal vinyl of the silane using conventional acrylate chemistry. The end result is a PEG that is covalently bonded to the metal surface. This reaction can be either light or peroxide initiated.

Accordingly, the present invention provides a treated surface exhibiting significant hydrophilic properties, with a surface contact angle of less than about 45 degrees, and preferably less than about 35 degrees. This treated surface has a low free energy and has provided demonstrated protein stability.

A variety of modifications and improvements to the present invention will be apparent to those skilled in the art. For example, it will be apparent that the invention can be applied to a wide range of different types of pump surfaces including metallic and non-metallic surfaces to reduce the surface contact angle for hydrophillic characteristics. Moreover, it will also be apparent that the invention can be applied to a broad scope of medical devices having a surface wherein avoidance of protein-based deposits is desired. Accordingly, no limitation on the invention is intended by way of the foregoing description and accompanying drawings, except as set forth in the appended claims.

## Claims

1. A medical device having a surface contactable by a protein-based medication, **characterised in that** a hydrophilic surfactant is covalently attached to said surface such that said surface has a surface contact angle less than 45 degrees, wherein said hydrophilic surfactant is a block ethylene/propylene copolymer.

2. The medical device of claim 1 wherein the hydrophilic surfactant has a molecular weight of about 1800 Daltons.

3. The medical device of claim 1 or 2 wherein the surface contact angle is less than 35 degrees.

4. The medical device of claim 1 or 2 wherein the surface contact angle is less than 10 degrees.

5. The medical device of claim 1 wherein the surface exhibits a protein adsorption profile of less than 1.0 microgram per square centimeter.

6. The medical device of claim 1 wherein the surface exhibits an insulin adsorption profile of less than 0.1 microgram per square centimeter.

7. The medical device of any one of the preceding claims wherein said surface is formed as part of a medication infusion pump.

8. The medical device of any one of the preceding claims wherein the surface is metallic.

9. The medical device of claim 8 wherein the surface is titanium.

10. The medical device of any one of claims 1 to 7 wherein the surface is non-metallic.

11. A method of treating a surface contactable by protein-based medication for use in a medical device, the method comprising covalently attaching to said surface a hydrophilic surfactant such that said surface has a surface contact angle less than 45 degrees, wherein said hydrophilic surfactant is a block ethylene/propylene copolymer.

12. The method of claim 11 wherein the hydrophilic surfactant has a molecular weight of about 1800 Daltons.

13. The method of claim 11 or 12 wherein the surface contact angle is less than 35 degrees.

14. The method of claim 11 or 12 wherein the surface contact angle is less than 10 degrees.

15. The method of claim 11 wherein the surface exhibits a protein adsorption profile of less than 1.0 microgram per square centimeter.

16. The method of claim 11 wherein the surface exhibits an insulin adsorption profile of less than 0.1 microgram per square centimeter.

17. The method of any one of claims 11 to 16 wherein the surface is metallic.

18. The method of claim 17 wherein the surface is titanium.

19. The method of any one of claims 11 to 16 wherein the surface is non-metallic.

20. The method of claim 11, wherein the hydrophilic surfactant is covalently attached to the surface by radiation, electron beam or photo-induced grafting, plasma polymerization, chemical grafting, plasma deposition or silane coupling.

## Patentansprüche

1. Eine medizinische Vorrichtung, die eine Oberfläche aufweist, die von einem Medikament auf Proteinbasis kontaktierbar ist, **dadurch gekennzeichnet, dass** ein hydrophiles oberflächenaktives Mittel derart kovalent an die Oberfläche gebunden ist, dass die Oberfläche einen Oberflächenkontaktwinkel von weniger als 45° aufweist, wobei das hydrophile oberflächenaktive Mittel ein Ethylen/Propylen-Blockcopolymer ist.

2. Medizinische Vorrichtung nach Anspruch 1, bei der das hydrophile oberflächenaktive Mittel ein Molekulargewicht von etwa 1800 Dalton aufweist.

3. Medizinische Vorrichtung nach Anspruch 1 oder 2, bei welcher der Oberflächenkontaktwinkel weniger als 35° beträgt.

4. Medizinische Vorrichtung nach Anspruch 1 oder 2, bei welcher der Oberflächenkontaktwinkel weniger als 10° beträgt.

5. Medizinische Vorrichtung nach Anspruch 1, bei der die Oberfläche ein Proteinadsorptionsprofil von weniger als 1,0 µg/cm² aufweist.

6. Medizinische Vorrichtung nach Anspruch 1, bei der die Oberfläche ein insulinadsorptionsprofil von weniger als 0,1 µg/cm² aufweist.

7. Medizinische Vorrichtung nach einem der vorstehenden Ansprüche, bei der die Oberfläche als Teil einer Medikamenteninfusionspumpe ausgebildet ist.

8. Medizinische Vorrichtung nach einem der vorstehenden Ansprüche, bei der die Oberfläche metallisch ist.

9. Medizinische Vorrichtung nach Anspruch 8, bei der die Oberfläche Titan ist.

10. Medizinische Vorrichtung nach einem der Ansprüche 1 bis 7, bei der die Oberfläche nicht-metallisch ist.

11. Ein Verfahren zur Behandlung einer Oberfläche, die von einem Medikament auf Proteinbasis kontaktierbar ist, zur Verwendung in einer medizinischen Vorrichtung, wobei das Verfahren das kovalente Binden eines hydrophilen oberflächenaktiven Mittels an die Oberfläche umfasst, und zwar derart, dass die Oberfläche einen Oberflächenkontaktwinkel von weniger als 45° aufweist, wobei das hydrophile oberflächenaktive Mittel ein Ethylen/Propylen-Blockcopolymer ist.

12. Verfahren nach Anspruch 11, bei dem das hydrophile oberflächenaktive Mittel ein Molekulargewicht von etwa 1800 Dalton aufweist.

13. Verfahren nach Anspruch 11 oder 12, bei dem der Oberflächenkontaktwinkel weniger als 35° beträgt.

14. Verfahren nach Anspruch 11 oder 12, bei dem der Oberflächenkontaktwinkel weniger als 10° beträgt.

15. Verfahren nach Anspruch 11, bei dem die Oberfläche ein Proteinadsorptionsprofil von weniger als 1,0 µg/cm² aufweist.

16. Verfahren nach Anspruch 11, bei dem die Oberfläche ein Insulinadsorptionsprofil von weniger als 0,1 µg/cm² aufweist.

17. Verfahren nach einem der Ansprüche 11 bis 16, bei dem die Oberfläche metallisch ist.

18. Verfahren nach Anspruch 17, bei dem die Oberfläche Titan ist.

19. Verfahren nach einem der Ansprüche 11 bis 16, bei dem die Oberfläche nicht-metallisch ist.

20. Verfahren nach Anspruch 11, bei dem das hydrophile oberflächenaktive Mittel durch strahlungs-, elektronenstrahl- oder photoinduziertes Pfropfen, eine Plasmapolymerisation, ein chemisches Pfropfen, eine Plasmaabscheidung oder eine Silankopplung kovalent an die Oberfläche gebunden wird.

## Revendications

1. Dispositif médical ayant une surface pouvant être en contact avec un médicament à base de protéine, **caractérisé en ce qu'**un tensioactif hydrophile est lié de façon covalente à ladite surface de telle façon que ladite surface ait un angle de contact de surface inférieur à 45 degrés, où ledit tensioactif hydrophile est un copolymère bloc éthylène/propylène.

2. Dispositif médical selon la revendication 1, où le tensioactif hydrophile a une masse moléculaire d'environ 1800 daltons.

3. Dispositif médical selon la revendication 1 ou 2, où l'angle de contact de la surface est inférieur à 35 degrés.

4. Dispositif médical selon la revendication 1 ou 2, où l'angle de contact de la surface est inférieur à 10 degrés.

5. Dispositif médical selon la revendication 1, où la surface présente un profil d'adsorption des protéines inférieur à 1,0 microgramme par centimètre carré.

6. Dispositif médical selon la revendication 1, où la surface présente un profil d'adsorption de l'insuline inférieur à 0,1 microgramme par centimètre carré.

7. Dispositif médical selon l'une quelconque des revendications précédentes, où ladite surface constitue une partie d'une pompe de délivrance d'une médication.

8. Dispositif médical selon l'une quelconque des revendications précédentes, où la surface est métallique.

9. Dispositif médical selon la revendication 8, où la surface est en titane.

10. Dispositif médical selon l'une quelconque des revendications 1 à 7, où la surface est non-métallique.

11. Méthode de traitement d'une surface pouvant être en contact avec un médicament à base de protéine utile dans un dispositif médical, la méthode comprenant l'attachement covalent à ladite surface d'un tensioactif hydrophile de telle façon que ladite surface ait un angle de contact de surface inférieur à 45 degrés, où ledit tensioactif hydrophile est un copolymère bloc éthylène/propylène.

12. Méthode selon la revendication 11, où le tensioactif hydrophile a une masse moléculaire d'environ 1800 daltons.

13. Méthode selon la revendication 11 ou 12, où l'angle de contact de la surface est inférieur à 35 degrés.

14. Méthode selon la revendication 11 ou 12, où l'angle de contact de la surface est inférieur à 10 degrés.

15. Méthode selon la revendication 11, où la surface présente un profil d'adsorption des protéines inférieur à 1,0 microgramme par centimètre carré.

16. Méthode selon la revendication 11, où la surface présente un profil d'adsorption de l'insuline inférieur à 0,1 microgramme par centimètre carré.

17. Méthode selon l'une quelconque des revendications 11 à 16, où la surface est métallique.

18. Méthode selon la revendication 17, où la surface est en titane.

19. Méthode selon l'une quelconque des revendications 11 à 16, où la surface est non-métallique.

20. Méthode selon la revendication 11, où le tensioactif hydrophile est attaché de façon covalente à la surface par greffage provoqué par une irradiation, un faisceau électronique ou un rayonnement photonique, une polymérisation par plasma, un greffage chimique, un dépôt par plasma ou un couplage de silane.
